# EUROPEAN PATENT SPECIFICATION

(11) **EP 3 856 011 B1**
(45) Date of publication and mention of the grant of the patent: **08.06.2022**
(21) Application number: 19773823.0
(22) Date of filing: 24.09.2019
(51) Int. Cl.: A61B 5/00, A61B 5/024, A61B 5/25

(54) **BODY MOUNTABLE SENSOR UNIT**
KÖRPERMONTIERBARE SENSOREINHEIT
UNITÉ DE DÉTECTION MONTABLE SUR UN CORPS

(30) Priority: 24.09.2018 EP 18196263
(43) Date of publication of application: 04.08.2021
(73) Proprietor: Koninklijke Philips N.V., 5656 AG Eindhoven (NL)
(72) Inventor: GELISSEN, Jozef Hubertus, 5656 AE Eindhoven (NL)
(74) Representative: Philips Intellectual Property & Standards
(86) International application number: PCT/EP2019/075689
(87) International publication number: WO 2020/064719

(56) References cited:
- EP-A1- 2 745 772
- WO-A1-2016/189422
- US-A1- 2018 146 870

## Description

### FIELD OF THE INVENTION

This invention relates to a body mountable sensor unit, in particular for use in monitoring one or more parameters of the human body.

### BACKGROUND OF THE INVENTION

EP 2 745 772 discloses a biosensor that includes light emitting elements and a light receiving element disposed on a principal surface of a wiring board; a light shielding portion disposed between a light-emitting-element sealing portion and a light-receiving-element sealing portion; a base medium having light transmitting properties, disposed in parallel with the wiring board with the light shielding portion therebetween; an adhesion layer having light transmitting properties, configured to bond the base medium with the light-emitting-element sealing portion, the light-receiving-element sealing portion, and the light shielding portion; and a first electrocardiograph electrode attached to a principal surface of the base medium. The refractive index of the base medium is set to be higher than that of the adhesion layer, and a surface of the first electrocardiograph electrode which is adjacent to the base medium is roughened so that stray light passing through the base medium will be scattered.

US 2018/0146870 discloses a device for sensing at least one biological parameter (e.g., heart rate, heart rate variability) of a subject, the device comprising a contact surface configured for being brought into a contact with a skin surface of the subject. The device has at least one light source for illuminating the skin surface through the contact surface along an illumination source optical axis with illumination including at least one sensing wavelength, and at least one detector for detecting a response of said illumination at least at said wavelength, from the skin surface through the contact surface along a detector optical axis, and providing signals configured for determining said biological parameter based thereon, said illumination optical axis forming with said contact surface an acute included angle.

WO 2016/189422 discloses an electrode body includes a conductive silicone material configured to enable uptake or diffusion of moisture from the skin of the subject over which the electrode body is disposed; and a detergent configured to facilitate a flow of ions through the conductive silicone material.

In the field of physiological parameter monitoring, there is a growing trend towards on-body systems, for example worn on the chest, for use both by hospitalized patients as well as discharged patients once at home. On-body units may for instance take the form of a skin-adhered patch. Different sensory modalities may be incorporated in an on-body unit.

Electrocardiography (ECG) and Photoplethysmography (PPG) are two sensory methods which can each be used to measure heart rate (for instance average heart rate or inter beat intervals). ECG utilizes the electrical stimulation of the heart muscle. PPG measures blood volume changes in the vascular bed beneath the sensor.

An ECG utilizes at least two electrodes, placed on the skin and records electrical activity of the heart over a period of time. The electrodes detect small electrical changes in underlying conductive tissue of the skin which arise due to the heart muscle's electrophysiological pattern of depolarizing and repolarizing during each heartbeat. This can be used to detect many physiological parameters including heart rate, and also respiration rate (through tracking changes in inter-beat intervals and/or modulation in the ECG signal for instance).

Photoplethysmography (PPG) is an optical measurement technique that evaluates a time-variant change of light reflectance or transmission of an area or volume of interest. PPG is based on the principle that blood absorbs light more than surrounding tissue, so variations in blood volume or blood pressure (e.g. with every heartbeat, respiratory motion, etc.) affect transmission or reflectance correspondingly. By detecting changes in detected blood-related signals, a cyclic signal corresponding to the pulse can be obtained.

A pulse oximeter is one exemplary application or function of a PPG-based sensor. While the purpose of such a sensor is to obtain a measure of blood oxygen saturation, it also detects changes in blood volume in the skin, and thereby performs PPG sensing. By detecting changes in blood volume, a cyclic signal corresponding to the optical pulse is obtained. Furthermore, this higher frequency pulse rate signal is modulated over a lower frequency signal which tracks the respiration rate. Thus, with suitable filtering of the PPG signal, both pulse rate and respiration rate signals can be obtained. PPG sensors may be thus commonly used to provide a measure of the pulse rate and respiration rate. Other applications of the detected PPG signal are also known, such as e.g. detection of atrial fibrillation, of sleep stages, of apnea, etc. by implementing appropriate data processing on the raw PPG signal.

Both ECG and PPG have attendant advantages and disadvantages.

Advantages of ECG include that it is universally clinically accepted as a measurement technique and that measurements are relatively unaffected by other physiological changes in the body. A disadvantage of ECG is that it requires at least two electrical contact points (by contrast, PPG requires only one point of (optical) interface with the body) and typically also a good interfacing with skin.

Advantages of PPG include that it requires only one point of contact with the body, and can be measured at almost any location on the body, making it highly flexible to different situations. Disadvantages of PPG however are that it is not universally clinically accepted as a physiological measure, and that measurements are somewhat affected by physiological changes, such as vasomotion.

It is known to include both ECG and PPG modalities in a single on-body measurement unit such as a wrist-worn device. However, known devices are large and cumbersome, since to include multiple electrodes of an ECG in addition to PPG optical components necessitates a significant overall surface area. This makes them uncomfortable and impractical for users, especially if worn long term. A further problem, for instance in the case of patch-based devices is that the large surface area prevents skin stretch. This is both uncomfortable, and reduces device lifetime, since if the patch area is too large it may detach from the skin more easily.

Furthermore, placement of the components requires careful consideration (especially in cases where sensor accuracy is of critical important, such as the professional clinical domain) since some components, such as the PPG optical sensor, require a surface clearance around them for blocking or intercepting incoming light (in the case of the PPG sensor) or for blocking ingress of dust or other contaminants. For some devices, such as patch-based units, space is also needed around certain components (such as the ECG electrodes) for adhesive to ensure proper contact is maintained with the skin. Ill-considered placement can diminish sensor accuracy which is of critical importance for professional clinical applications (as opposed for instance to mainstream consumer electronics applications).

An improved on-body sensor device is sought, which is more comfortable and less cumbersome when worn by a user, but wherein sensor accuracy is not reduced.

### SUMMARY OF THE INVENTION

The invention is defined by the independent claim. The dependent claims define advantageous embodiments.

According to examples in accordance with an aspect of the invention, there is provided a body-mountable sensor unit, comprising:
a skin-contact surface for engaging with skin of a user when the unit is in a body-mounted configuration;
a PPG sensor, comprising at least one light source, adapted to generate light of a particular spectral composition, and at least one optical detector, sensitive to at least a portion of said spectral composition, the light source and optical detector arranged to emit light to and receive light from the skin in contact with the skin-contact surface; and
an ECG sensor, the ECG sensor comprising at least one electrode, being electrically exposed at the skin-contact surface;
a section of the skin contact surface being defined by a skin contact surface element, the element having at least a portion arranged between the optical detector and the at least one light source of the PPG sensor, wherein said surface element is adapted to be absorptive of at least a majority of said spectral composition, and
wherein a first of said at least one electrode is located at, or forms in, at least a part of, said surface element, the electrode being adapted to be absorptive of at least a majority of said spectral composition.

Embodiments of the invention are based on minimizing the total unit surface space by efficiently sharing certain surface regions for multiple functions. Diminution of sensor accuracy is avoided through integration of certain light absorbing material sections.

For optimal PPG sensor accuracy, a light-absorbing surface element is provided having at least a portion arranged between the optical detector and the at least one light source, for example interposed between the detector and light source. This is to prevent direct light penetration from the light source to the optical detector, which can interfere with readings. This section is hence absorptive of at least a majority of the spectral composition of the light which is generated by the light source.

The surface element is hence a light blocking element for instance.

Since this section of the skin contact surface should contact the skin in use, but has no further sensing functionality, it has been realized by the inventor that this region may usefully be utilized for inclusion of an ECG electrode. Reduction of the important light-blocking effect of the surface element at this section is avoided by ensuring that the electrode (e.g. at least a skin contacting surface part of the electrode) is also absorptive of at least a majority of the spectral composition which is generated by the at least one PPG light source.

In this way, a total skin-contact surface area of the sensor unit can be reduced, without diminishing accuracy of the PPG sensor readings. Hence this permits an overall form factor of the unit to be reduced, without compromising the reliability of the unit for physiological parameter monitoring in for example the professional clinical domain.

The first electrode may be located at the surface element. This may mean for example located co-incident with the surface element, or within the outer bounds of surface element. It may be disposed atop the surface element or arranged surrounded by the surface element for example.

In further examples, the first electrode may form at least a part of the surface element. It may form the whole of the surface element.

For the purposes of the present disclosure, reference to light is taken to include reference to infrared light and other non-visible portions of the electromagnetic spectrum.

The surface element is adapted to be absorptive of at least a majority of the spectral composition of light generated by the at least one PPG sensor light source. By this is meant at least 50% of the spectral composition, preferably at least 60% of the spectral composition, more preferably at least 70% of the spectral composition, and even more preferably at least 80% of the spectral composition.

The spectral composition of the light refers to the component wavelengths (or frequencies) of electromagnetic radiation comprised by the light.

The surface element provides the function of preventing light generated by the at least one light source from reaching the optical detector without travelling through the skin, i.e. preventing direct penetration of the light to the detector without any intermediate absorbance by or reflection on the skin.

The surface element may in some cases be raised or elevated relative to surrounding areas of the skin contacting surface to enhance interception of light propagating directly from the at least one light source. It may be raised relative to the optical detector so as to restrict the range of angles from which light can propagate directly into the optical detector. In particular, light travelling transverse or parallel with the skin-contact surface may be blocked or intercepted by the surface element, and absorbed by the light absorbing surface.

In advantageous examples, the surface element may extend depth-wise into a body of the unit for intercepting or blocking light penetrating through the body between the at least one light source and the optical detector.

For maximal light-intercepting effect, said surface element may be an annular surface element, arranged extending around at least the PPG optical detector and/or the at least one light source.

For simplicity of construction, and to maximize the light-blocking effect, said first electrode of the ECG may be formed of a material being absorptive of at least a majority of said spectral composition (of the light which the at least one light source is adapted to generate). The color of the material may at least partially facilitate this absorption for instance. In this way, a light-absorbing exposed surface color is automatically provided by the material composition of the electrode for example.

For example, said first electrode may comprise or be formed of a material comprising:
a conductive silicone material configured to enable uptake or diffusion of moisture from the skin of a user in which the electrode is placed in contact,
electrically conductive particles, the particles including at least electrically conductive carbon particles, and
a detergent configured to facilitate a flow of ions through the conductive silicone material.

This example material provides a dry ionic electrode material. Due to the inclusion of the carbon particles, such a material can be easily formed of a dark, light-absorbing color, such as black or grey, to facilitate the above feature. The carbon also enhances conductivity.

This example material is described in detail in WO 2016/189422. All options and variations for the electrode material outlined in the description and claims of this application may be applied also to the first electrode of embodiments of the present invention.

As noted above, a first of the at least one electrode is located at or forms at least a part of said surface element.

In the case the first electrode is merely located at the surface element, this means the surface is located at, for example exposed at, a region of the skin contact surface falling within the bounds of said surface element, i.e. located within the bounds of said section of the skin contact surface defined by the surface element. It may be disposed atop the surface element in some cases, or arranged encompassed or surrounded by it.

In the case the first electrode forms at least part of the surface element, it may fully form the surface element, i.e. the exposed surface of the electrode may provide said section of the skin contact surface defined by the surface element. In other cases, it may form only a part of the surface element, i.e. provide only that portion.

As noted above, at least a portion of the surface element is arranged between the optical detector and the at least one light source of the PPG sensor.

The at least portion of the skin contact surface element may span a distance between the at least one optical detector and the at least one light source, i.e. may extend a full distance from the at least one optical detector to the at least one light source.

According to advantageous examples, the surface element may extend beyond the at least one light source.

For example, the surface element may extend away from the optical detector to a point or a line between the at least one light source and a periphery of the skin contact surface.

The surface element may thereby encompass the at least one light source, i.e. so that an optical output area of the at least one light source is located within or encompassed by the surface element.

The surface element may be an annular surface element, arranged extending around the PPG optical detector.

According to any of the above examples, the surface element and/or the exposed surface of the first electrode may be black or grey, e.g. formed of a material being black or grey in color. The color black or grey is absorptive of light of all wavelengths. The particular shade of grey or black (from light grey, to full black) simply configures the quantity or proportion of incident light which is absorbed. Lighter grey absorbs a smaller proportion of incident light than darker grey and black. For optimum light absorption, the surface element may be black.

According to one or more embodiments, the body-mountable sensor unit may further comprise a light-blocking ring extending around at least the at least one light source and optical detector, the ring protruding from the skin-contact surface and for engaging with skin of a user in use to block passage of ambient light. The light blocking ring may form a skirt or protruding lip for example which engages in a sealing fashion with the skin for blocking passage of light. The ring may be a light-sealing ring.

The light blocking ring may extend around a peripheral region of the skin-contact surface. A peripheral region means a region closer the periphery of the skin-contact surface than the center or middle. For example, the peripheral region may be an outer annular region extending radially between anywhere from mid-way between a center point and an outer-most periphery and the outer-most periphery, e.g. anywhere from a radial mid-point of the skin contact surface and the outer periphery.

According to one or more examples, the light-blocking ring may carry at least a further electrode of the ECG sensor, the further electrode being electrically exposed at a skin-engaging surface of the ring. The further electrode may for example be integrated or partially embedded in the ring or may be carried on a surface of the ring. This provides a compact spatial placement for the further electrode, since the same skin-contact area is utilized for the light-blocking element as for ECG electrode contact.

According to any embodiments, the optical detector of the PPG sensor may advantageously be disposed in a central region of the skin contact surface. A central region may mean a region closer the center of the surface than an outer periphery. This region may extend for instance anywhere from a mid-point between the center and an outer periphery, and the center.

By locating the optical detector in a central region, it is as remotely located from the periphery as possible. This minimizes penetration of any ambient light (which may otherwise leak from the periphery) through to the optical detector. This improves accuracy of measurements.

The at least one light source may then be disposed at a location between the optical detector and a periphery of the skin contact surface in some examples.

According to one or more examples, the sensor unit may include a plurality of light sources, arranged around the optical detector. The at least portion of the skin contact surface element may extend annularly around the optical detector, and may include a portion between each of the light sources and the optical detector.

According to one set of embodiments, the body-mountable sensor unit may be in the form of a body-mountable or wearable patch. The body-mountable unit may in this case for instance be in the form of a flexible sheet or element, having an adhesive layer across at least a portion of a skin contact surface, for use in adhering the patch to a user's skin. The patch may comprise a textile layer. In other examples, the patch may comprise a foam material for cushioned application to skin.

According to a further set of embodiments, the body mountable unit may be in the form of a wearable device, for example a wrist-worn device. This may be a smart watch device. In this case the body mountable unit may include a main body portion having the skin contact surface, and this body portion including or being partially formed by the PPG sensor and ECG sensor. This body portion may be coupled to a strap arrangement for mounting the body portion removably to a user's wrist.

The body-mountable sensor unit may be for monitoring one or more physiological parameters. The unit may be a physiological parameter monitoring unit.

According to any embodiment, the body-mountable sensor unit may further comprise a controller adapted to receive signal outputs from the at least one electrode and from the optical detector, and to derive a measure of one or more physiological parameters based on the signal outputs.

The one or more physiological parameters may include a heart rate. The one or more physiological parameters may additionally or alternatively include a blood oxygen saturation level (SpO₂), inter-beat interval rate and/or a respiration rate.

The physiological parameters may additionally or alternatively include blood pressure or blood pressure related metrics such as pre-ejection phase, pulse transit time, pulse arrival time. These measures are derivable for example from a combination of the PPG and ECG sensor signals.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 shows an example on-body sensor unit according to one or more embodiments of the invention;
Fig. 2 shows a cross-section through a further example on-body sensor unit according to one or more embodiments;
Fig. 3 shows a further example on-body sensor unit according to one or more embodiments; and
Fig. 4 depicts a layout of a further example on-body sensor unit according to one or more embodiments.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides an on-body sensor unit comprising a PPG sensor and an ECG sensor, each having sensor parts exposed at a skin-contact surface of the unit. The PPG includes at least one optical detector and at least one light source. Disposed between the at least one light source and the optical detector is a light-intercepting surface element which is absorptive of at least a majority of a spectral composition of the light which is generated by the PPG light source. Located at, or forming at least a part of, this surface element is an electrode of the ECG sensor, said electrode being adapted to be absorptive of a majority of the spectral composition of said light generated by the at least one light source.

The invention makes use of a PPG sensor. The basic principles of PPG will now be briefly outlined.

Photoplethysmography (PPG) is an optical measurement technique that evaluates a time-variant change of light reflectance or transmission of an area or volume of interest. PPG is based on the principle that blood absorbs light more than surrounding tissue, so variations in blood volume with every heartbeat affect transmission or reflectance correspondingly. By detecting changes in blood volume, a cyclic signal corresponding to the optical pulse is obtained.

In addition to information about the heart rate, a PPG waveform can comprise information attributable to other physiological phenomena such as respiration. By evaluating the transmittance and/or reflectivity at different wavelengths (typically red and infrared), the blood oxygen saturation can also be determined.

A PPG sensor for example contains at least one LED, and one light detector. The LED and detector are placed such that the LED directs light into the skin of the user, which is reflected or transmitted, and detected by the detector. The amount of reflected/transmitted light is determined by, amongst others, the perfusion of blood within the skin.

The PPG system for example includes a red LED, a near-infrared LED, and a photodetector diode. The PPG sensor is typically configured with the LEDs and photodetector diode directly on the skin of the patient, typically on a digit (finger or toe) or earlobe.

Other places on the patient may also be suitable, including the forehead, the nose or other parts of the face, the wrist, the chest, the nasal septum, the alar wings, the ear canal, and/or the inside of the mouth, such as the cheek or the tongue.

More than one LED can be provided. The LEDs may emit light at different wavelengths, which light is diffused through the vascular bed of the patient's skin and received by the photodetector diode. The changing absorbance at each of the wavelengths is measured, allowing the PPG sensor (or an additional signal processing unit connected to the detector of the PPG sensor) to determine the absorbance due to the pulsing arterial blood alone, excluding venous blood, skin, bone, muscle, and fat for example. The resulting PPG signal may then be analyzed.

In transmissive pulse, a sensor device is placed on a thin part of the patient's body. Reflectance pulse may be used as an alternative to transmissive pulse. This method does not require a thin section of the person's body and is therefore well suited to more universal application such as the feet, forehead and chest.

When integrating a PPG and ECG in a single sensor unit, there are a large number of demands on surface area space. This is particularly the case where accuracy of the sensor measurements is of critical importance (such as in the professional medical domain), since here, additional elements may be needed for light blocking.

For example, in certain preferred cases, when integrating a PPG and ECG sensor in a single body-mountable unit, such as a patch, each sensor modality (e.g. ECG electrode and PPG optical detector) requires at least a first area for the sensor (e.g. approximately 1-2 cm diameter) as well as preferably a ringed area (e.g. approximately 1cm width) around the respective sensor for making contact with the skin.

In the case of an ECG sensor, this ringed area may for example be an adhesive ring for coupling the sensor flush against the user's skin, and for preventing ingress of moisture, dust and other contaminants. In the case of a PPG optical detector, this ringed area may be for performing a light intercepting function. In particular, it is preferably to have a section of the skin-contacting surface between the PPG optical detector and light source defined by a body or element which is light-absorptive for impeding passage of light directly from the PPG light source to the optical detector.

Hence, in each case, the ringed area is for improving accuracy or reliability of the sensor.

As discussed above, for on-body sensor units, reducing a total required skin contact area of the device is of benefit in terms of comfort and practicality for a user. In particular, this allows a form factor of the device to be reduced, rendering it less cumbersome, and, in the case of patch-based devices, allowing more space for adhesive, so improving adherence and hence lifetime of the patch.

Embodiments of the invention provide a body-mountable sensor unit having both PPG and ECG sensors wherein a total skin contact area is reduced by integrating sensing and light-blocking functionality within certain shared surface sections.

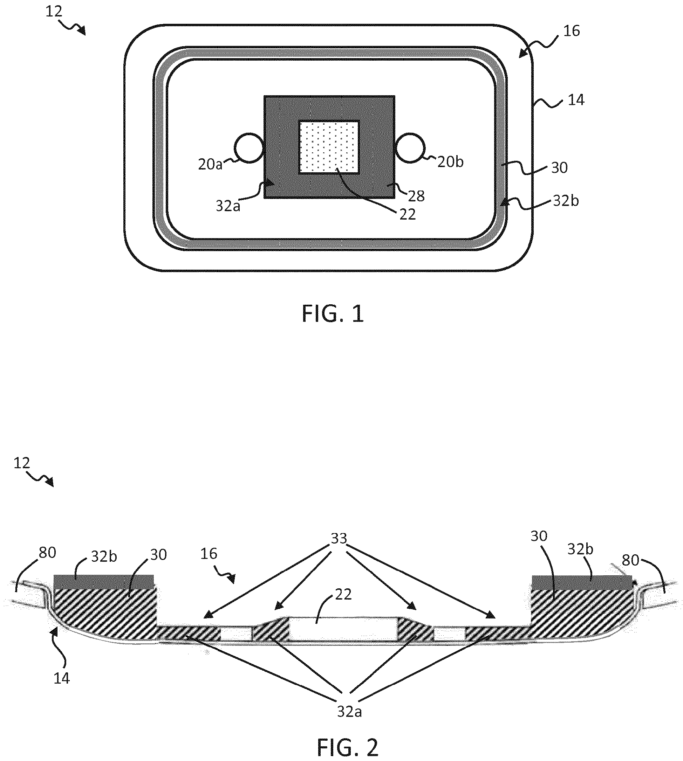

Fig. 1 schematically depicts an example body-mountable sensor unit 12 in accordance with an embodiment of the invention. The example sensor unit shown is in the form of a patch. However, this represents just one example, and the descriptions, explanations and variations outlined below may be applied with equal advantage to any other form of body-mounted unit, e.g. a wearable device such as wrist-worn device.

The body-mountable sensor unit 12 comprises a body portion 14, having a skin-contact surface 16 for engaging with skin of a user when the unit is in a body-mounted configuration. Fig. 1 hence shows an 'underside' of the unit, which is applied to a skin region of a user when the unit is in a mounted configuration.

In the patch example of Fig. 1, the body portion comprises a thin, flexible sheet or pad element (i.e. a patch), which may for example be at least partially comprised of a textile material or a flexible plastic material. All or part of the skin contact surface 16 of the body portion may be coated with an adhesive layer for adhering the unit to the skin of a user in use.

The skin-contact surface refers to the exposed surface of the unit which makes contact with the skin of a user when the unit is mounted to the body. It is at least partially formed by an exposed outer surface of the sheet or patch element, and may further comprise the exposed surfaces of elements coupled or mounted to the sheet or patch.

Included within the body portion 14 is a PPG sensor, comprising two light sources (20a, 20b), adapted to generate light of a particular spectral composition, and an optical detector 22 being sensitive to said spectral composition. More than two light sources may alternatively be provided, or a single light source may be provided in other examples. More than one optical detector may also alternatively be provided in further examples.

The light sources 20a, 20b and optical detector 22 are arranged to emit and receive light respectively at the skin-contact surface 16. In particular, each comprises a respective optical window exposed at the skin-contact surface for emitting or receiving light respectively. For example, each light source 20a, 20b has a light output surface arranged at the skin contact surface for emitting light in use. The optical detector 22 has a light input window arranged at the skin-contact surface for receiving light reflected from the incident skin in use.

In one or more examples, the light input window of the optical detector 22 comprises a light guide or lens for guiding light reflected from the skin into the optical detector during use of the device. The light guide or lens may protrude from the skin contacting surface 16. It may convexly protrude.

Each of the light sources 20a, 20b, may by way of example comprise one or more LEDs. The light source may be adapted to generate light of any spectral composition. This may include visible light but also infrared light or other non-visible portions of the electromagnetic spectrum. Typically for a PPG, red light is generated by the light sources. The optical detector may be or comprise a photodetector diode.

To minimize penetration of ambient light to the optical detector 22, the detector is located at a central region of the skin contacting surface 16. The two light sources are positioned at either side of the optical detector, between the detector and periphery of the skin contact surface.

The body portion further includes an ECG sensor, the ECG sensor comprising two electrodes 32a, 32b, each arranged electrically exposed at the skin-contact surface 14. More than two electrodes may be provided in alternative examples. In other examples, only one electrode may be included in the unit. Although an ECG sensor typically requires inputs from two electrodes to function, a second electrode may for example be separately provided, with the ECG sensor arranged operably coupleable with such an electrode for example.

The skin contact surface 16 includes a surface element 28 disposed between the light sources 20a, 20b and the optical detector 22 of the PPG, this surface element being adapted to be absorptive of light of at least a majority of the spectral composition which is generated by the light sources 20a, 20b. The surface element defines a section of the skin contact surface between the light sources and the optical detector.

A majority means at least 50%, and more preferably at least 60%, or at least 70% or at least 80% for instance. For example, it may be formed of a material having a color which is absorptive of light of said spectral composition. In this way, the surface element performs a light impeding function, acting to at least partially absorb light emitted by the light sources in the direction of the PPG sensor.

The surface element 28 impedes or blocks passage of light directly from the light sources 20a, 20b to the optical detector, 22, which light can interfere with detector readings. PPG sensor accuracy and reliability is thereby enhanced.

More specifically, the surface element is arranged for preventing light generated by the light sources from reaching the optical detector without travelling through the skin, i.e. preventing direct penetration of the light to the detector without any intermediate absorbance by or reflection by the skin.

The surface element 28 may be a layer element, and may have a body which extends depth-wise into the body portion 14 of the sensor unit in some examples. In this way, the surface element may be arranged to block light from penetrating through the body portion between either of the light sources and the optical detector.

In the example of Fig. 1, this surface element 28 is black or grey. However, other colors absorptive of said spectral composition may alternatively be used. Furthermore, in the example of Fig. 1, the surface element is an annular surface element and extends around the optical detector 22 of the PPG.

A typical minimum distance between the PPG optical detector 22 and each of the light sources 20a, 20b may be between 5 and 8 mm according to certain examples. This is exemplary only and the inventive concept is not limited to such dimensions. In these cases, the light absorbing surface element may have a minimum radial extension of between 5 and 8 mm. For example, the surface element may be an annular surface element having an annular ring width of between 5 mm and 8 mm.

To efficiently conserve space, a first 32a of said electrodes of the ECG is arranged so as to provide, i.e. form said light-absorbing surface element 28, with the electrode being absorptive of light of at least a majority of said spectral composition generated by the light sources 20a, 20b. A color of a material forming the electrode may at least partially facilitate this. A color of the electrode material, e.g. a material forming at least an exposed surface of the electrode, may be black or grey.

In particular, in this example the first electrode is arranged in the body portion such that its exposed surface extends between the two light sources 20a, 20b and the optical detector 22. The electrode thus forms the light-absorbing surface element defining one portion of the skin contact surface 16, and being absorptive of light of at least a majority of the spectral composition generated by the light sources 20a, 20b (for instance having a color being so absorptive). The first electrode 32a is in particular provided in an annular shape to thereby provide an annular surface element.

Although in this example, the light blocking surface element spans the distance between the PPG optical detector and the light sources, in other examples, it may cover only a portion of this distance. It may be arranged simply interposed between the optical detector and the light sources for example.

As noted above, the first electrode 32a is adapted to be absorptive of light of at least a majority of said spectral composition. For example, it has at least an exposed surface which is absorptive of light of said spectral composition.

In some cases, the electrode 32a may be provided with a conductive coating or skin on its exposed surface, this coating or skin being absorptive to light of at least a majority of said spectral composition. A color of the coating or skin may facilitate this for instance.

In other examples, the electrode 32a may be formed or composed (entirely) of a material which is absorptive of at least a majority said spectral composition. This provides for a structurally simpler arrangement, since the material composition of the electrode itself provides for the light absorptive property.

According to any embodiment of the present invention, the electrode may be formed of a material comprising:
a conductive silicone material configured to enable uptake or diffusion of moisture from the skin of a user in which the electrode is placed in contact,
electrically conductive particles, the particles including at least electrically conductive carbon particles, and
a detergent configured to facilitate a flow of ions through the conductive silicone material.

Implementation of this material is described in detail in the description and claims of WO 2016/189422. All options and variations for the electrode material outlined in the description and claims of this application may be applied also to the first electrode of embodiments of the present invention.

A non-limiting selection of implementation options for the electrode material will now be outlined.

The electrode may be formed of a material comprising a silicone material, electrical conductive particles, and detergent. In some examples, in addition to carbon, the electrical conductive particles may include graphite, and/or silver flakes or particles.

In some examples, the electrical conductive particles may be mixed with the silicone material to form a conductive silicone material. In some examples, the electrode material may include a range of about 25 to 75 % weight of the silicone material, a range of about 20 to 50 % weight of the conductive particles and a range of about 5 to 25 % weight of the detergent. In some examples, the electrode material may include 50 % weight of the silicone material, 35 % weight of the conductive particles and 15 % weight of the detergent. In some examples, the electrode material may be formed by mixing 15g of the detergent and 35g of the conductive particles with 50g of the silicone material.

In some examples, the silicone material, the conductive particles and the detergent may be mixed using mix-extrusion, high speed mixing process, which may be performed at temperature ranges of about 10 to 150°C. In some embodiments, the silicone material, the conductive particles and the detergent may be mixed using a high speed mixing or mix extrusion device.

In some examples, the conductive silicone material may be configured to provide moisture regulating properties to the first electrode. In some embodiments, the conductive silicone material may be a hydrophilic conductive silicone material or a PolyDiMethylSiloxane material. The added hydrophilic properties of the conductive silicone material cause moisture uptake from the subject's skin and from the air surrounding the subject's skin. Thus, the conductive silicone material of the electrode provides a comfortable layer against the skin. In some embodiments, the conductive silicone material may include electrically conductive liquid silicone rubber. In some embodiments, the conductive silicone material may include commercially available Elastosil^{®} LR 3162 material.

In some examples, the soap or detergent may include alpha- olefin sulfonate, soap, Sodium (C14-16) olefin sulfonate, and olefin sulfonate. In some examples, the soap or detergent are mixed with the conductive silicone material to form a homogeneous material. In some embodiments, the detergent may not only be mixed with the silicone material, but also chemically bonded onto the backbone of the silicone (e.g., PolyDiMethylSiloxane) material. In some embodiments, the soap or detergent may include commercially available Bio- Terge^{®} AS-90 Beads. In some embodiments, 9% of detergent (e.g., Bio-Terge^{®} AS-90 Beads) may be added to the conductive silicone material (e.g., Elastosil^{®} LR 3162).

In some examples, the detergent is configured to absorb water into the silicone material. In some embodiments, the detergent is configured to absorb more than 20 % by weight of water. The interaction of water with the ions (added by the detergent) is configured to function as a salt-bridge. In some embodiments, the interaction of water and/or bodily/body fluids with the ions (added by the detergent) is configured to function as an ion-richer electrolyte- electrode bridge.

In some examples, the detergent additives which are mixed into the conductive silicone material may be configured to improve the ion flow through the conductive silicone material. This improves the electrode-electrolyte interface of the dry silicone electrode. In some embodiments, the electrode is configured to enable current to pass across the electrode-electrolyte interface. In some examples, the detergent added to the silicone material may have either a liquid form or a powder/solid form.

In some examples, the silicone material may be filled with the conductive particles (at least carbon and optionally also graphite and/or silver for instance) to obtain electrical properties. In some embodiments, the conductive particle added to the silicone material may have either a liquid form or a powder/solid form. This makes the material conductive for electrons but not for ions. By adding ions into the conductive silicone material, two properties change in favor of lowering the impedance of the electrode path in interaction with the skin. First, the exchange of ions becomes possible and the modified silicone is configured to absorb/drag water from sweat in contact with the skin. In some embodiments, water uptake is not only from the skin but also from e.g. humidity of air, tap water, humid storage packaging, and/or showering. The absorption of water is configured to work as a salt-bridge. The silicone may in this way be modified into a bulk hydrophilic compound or a bulk ionic conductive hydrophilic compound.

In some examples, the first electrode 32a may have a body which extends (depth-wise) down into the body portion 14 of the body-mountable sensor unit 12, or forms a part of this body portion. This sunken portion may then provide a further light blocking effect, inhibiting potential penetration of light through the material of the body portion, between the PPG optical detector 22 and the light sources 20a, 20b. Such an example will be described below.

Although in the arrangement illustrated in Fig. 1, the surface portion 28 is formed by the first electrode 32a, this is not essential. In alternative examples, the electrode may be located at or within a broader light absorbing surface element 28. The first electrode may in this case form only one portion of the surface element 28 or may be mounted atop and extend over a portion of the light-absorbing surface element.

The example body-mountable sensor unit 12 of Fig.1 further comprises a light-blocking ring 30 arranged extending around a periphery of the body portion 14. In this way the light blocking ring extends around or encompasses the light sources 20a, 20b and the optical detector 22 of the PPG. The ring protrudes from the skin-contacting surface 14 such that when the body mounted sensor unit 12 is mounted to the body, the ring engaging sealingly with the incident skin of a user to thereby block passage of ambient light through to the PPG optical detector 22.

In the particular example of Fig. 1, to further conserve space, the light blocking ring carries the further electrode 32b of the ECG sensor, the further electrode being electrically exposed at a skin-engaging surface of the ring. The further electrode may be integrally included in the ring, or may be mounted or coupled to a skin-engaging surface of the ring.

In use, the body-mountable sensor unit 12 is attached or mounted to the body with the skin contact surface applied engagingly with the subject's skin. In the case of the patch embodiment shown in Fig. 1, the patch is adhered to the patient's skin with the skin-contacting surface applied against the skin. A layer of adhesive across one or more portions of the skin-contacting surface 16 acts to adhere the patch to the skin. Other means of adhering the patch to the skin as will be apparent to the skilled person may alternatively be used.

With the body-mountable sensor unit 12 mounted to the body, the ECG electrodes are arranged in electrical contact with the skin, and the PPG light sources 20a, 20b and optical detector 22 are arranged in optical communication with the skin.

The sensor unit 12 may further comprise a controller (not shown), arranged to receive signal outputs from each of the electrodes and from the optical detector, and to derive a measure of one or more physiological parameters based on the signal outputs. The controller may further control the light sources and the optical detector of the PPG.

More particularly, in use, the light sources 20a, 20b may be controlled to direct light into the skin of the user, which is reflected or transmitted, and detected by the optical detector 22. The amount of reflected/transmitted light is determined by, amongst others, the perfusion of blood within the skin.

The incident light is diffused through the vascular bed of the patient's skin and received by the optical detector. The changing absorbance of the light may be measured, allowing the detector (or a signal processing unit connected to the detector 22) to determine the absorbance due to the pulsing arterial blood alone, excluding venous blood, skin, bone, muscle, and fat for example. The resulting PPG signal may then be analyzed.

The light absorbing surface element 28 between the light sources 20a, 20b and the optical detector 22 ensures that substantially all light entering the optical detector is light reflected from the skin and not light received directly from the light sources 20a, 20b. Light propagating directly from the light sources is intercepted by the surface element 28 and is substantially absorbed.

The surface element 28 may be raised or elevated relative to surrounding areas of the skin contacting surface to enhance interception of light propagating directly from the light sources 20a, 20b.

Fig. 2 shows a cross-sectional view through a further example body-mountable sensor unit 12 in accordance with one or more embodiments. The sensor unit in this case is illustrated in the form of a wrist-mountable device, having a body portion 14 and coupled straps 80 for releasably mounting the body portion to a user's wrist. However, the described arrangement of sensor components for this example may be applied to a body-mountable unit of any type including for instance a body-mountable patch unit. Any of the above options or variations described in relation to the example of Fig. 1 may be applied also for the presently described example.

As in the example of Fig. 1, the sensor unit 12 comprises a body portion 14 having a skin contact surface 16. Comprised within or as part of the body portion is a PPG sensor having an optical detector 22 arranged substantially centrally within the skin contact surface 16, and two LED light sources 20a, 20b arranged at either side of the optical detector.

The optical detector is optically exposed at the skin contact surface, in particular having a light input window exposed at the skin contact surface 16.

The light sources 20a, 20b are each optically exposed at the skin contact surface, each having light output windows optically exposed at the skin contact surface 16. Each of the light sources is adapted in use to generate a light output have a defined spectral composition.

Also comprised by the body portion 14 is an ECG sensor having two electrodes (32a, 32b).

A first of the electrodes 32a is in this example formed of a material being electrically conductive and being absorptive of light of at least a majority of the spectral composition as is generated by the light sources 20a, 20b. This first electrode is arranged such that an electrically exposed surface 33 of the electrode 32a extends from an outer periphery of the PPG optical detector 22, and to, and beyond, the LED light sources. It extends to a point between the light sources and a periphery of the skin contact surface. In this way the exposed surface of the electrode encompasses or surrounds the light sources. The exposed surface in this example is annular and fully surrounds and encompasses both the optical detector 22 and the light sources 20a, 20b.

Due to the fact that the material of the first 32a electrode is absorptive of light of the same spectral composition as is generated by the light sources 20a, 20b, it forms the surface element being absorptive of light of this spectral composition.

As can be seen in Fig. 2, a body of the first electrode 32a extends depth-wise into the body portion 14 of the sensor unit 12 and thereby partly forms or constitutes a body of the body portion.

The body mountable sensor unit 12 further includes a light blocking ring 30 extending around a periphery of the skin contact surface 16. This protrudes from the skin contact surface for making (light) sealing engagement with the skin of a user when the unit is mounted to the wrist. This helps to prevent penetration of light beneath the body portion 14, where it may interfere with the optical detector.

The light absorbing surface element formed by the first electrode 32a extends from the PPG optical detector 22, beyond the light sources 20a, 20b and up to the light blocking ring 30.

The second electrode 32b is mounted electrically exposed at a skin-contact surface of the light blocking ring, as in the example of Fig. 1.

The PPG optical detector 22 protrudes slightly at the skin contact surface. This is due to a protruding transparent cover provided over the optical detector. This acts as an optical window for the optical detector. It may be adapted to act a lens element: focusing, directing or otherwise optically processing light in advance of its receipt within the optical detector 22. Such a protruding optical window is not essential however and may be omitted in other examples.

Although in this example, the light blocking first electrode spans a full distance from the PPG optical sensor to the light blocking ring (i.e. bridges this distance), this is not essential. It may be arranged only between the optical detector and light sources for example. It may be arranged to at least interpose the PPG optical detector and the light sources for example.

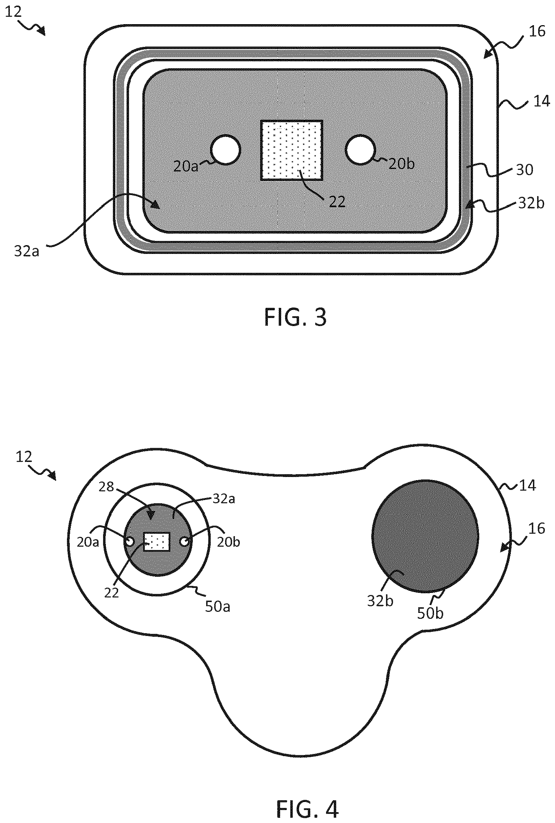

By way of further example, Fig. 3 illustrates a further example body mountable sensor unit 12. This example is shown in the form of a body-mountable patch unit. However, the same configuration of sensor components may be applied to a body-mountable unit of any form, including for instance a wearable device.

This illustrated example is similar to the example of Fig. 2, comprising a first ECG electrode 32a which extends from an outer periphery of the PPG optical detector 22 and up to and beyond the light sources 20a, 20b disposed at either side of the PPG optical detector. In this way the exposed surface of the electrode encompasses the light sources.

The exposed surface in this example is annular and fully surrounds both the optical detector 22 and the light sources 20a, 20b. The first electrode is shown stopping slightly short of the inner perimeter of the light blocking ring 30. However, the electrode may alternatively extend all the way to the inner perimeter of the light blocking ring.

As in the example of Fig. 2 above, said first 32a electrode is formed of a material being electrically conductive, and being absorptive of light of a majority of the spectral composition generated by the light sources 20a, 20b.

The second electrode 32b is again arranged electrically exposed at a skin-contact surface of the light blocking ring, as in the example of Fig. 1 and 2.

The skin contact surface 16 of the body portion 14 may comprise an adhesive layer coating for adhering the patch to a user's body in use.

Fig. 4 shows by way of example the layout of a further example body-mountable sensor unit 12. The body mountable sensor unit comprises a PPG sensor and an ECG sensor. As in the examples of Fig. 1 and Fig. 3, this unit is in the form of a body-mountable patch.

The patch comprises a body portion 14 with a skin contacting surface 16. Included on the skin contacting surface are two round pad elements 50a, 50b, spatially separated from one another, the pad elements for engaging with the skin of the user in use. Exposed at a surface of a first of the pad elements are an optical detector 22 and two light sources 20a, 20b of a PPG sensor. An electrically exposed surface of a first electrode 32b of an ECG sensor forms an annular light-absorbing surface element 28 extending between the optical detector 22 and a point just beyond the light sources 20a, 20b. This light absorbing surface element thereby encompasses and surrounds the light sources 20a, 20b.

In alternative examples, the exposed surface of the first ECG electrode 32a may extend to cover a larger area than that shown in Fig. 1, or may cover a smaller area. At minimum, the light absorbing surface element 28 formed by the ECG electrode 32a should have at least a portion arranged interposed between the optical detector 22 and the light sources 20a, 20b.

The features and options described in relation to the light sources 20a, 20b, optical detector 22 and the annular first electrode 32a of any of the above examples of Fig. 1, Fig. 2 or Fig. 3, may be applied also to these elements in the example of Fig. 4.

Exposed at a skin contact surface of the second pad element 50b is a second ECG sensor electrode 32b. This electrode covers the whole surface area of the second pad element 50b. However, this is by way of example only. In further examples, the second electrode exposed surface may cover a smaller area.

In general, a larger surface area for either the first or second ECG electrode is advantageous, since ECG sensor sensitivity is improved.

The spatial separation of the two ECG electrodes is hence greater in the present example of Fig. 4 than in that of Fig. 1. This may be preferable for instance in cases where placement of ECG electrodes on either side of the heart is desired. The patch of Fig. 4 may be attached for example to the chest, with the two pad elements 50a, 50b applied against respective regions of the skin positioned at either side of a subject's heart. For example, the two pad elements may be at least 5 cm or at least 10 cm apart.

The skin contact surface 16 may comprise an adhesive layer coating for adhering the patch to a user's body in use. The pad elements may be at least partially coated with an adhesive layer for adherence of the electrodes and PPG components to the body.

Although both embodiments described in detail above relate to patch-based units, other body-mountable units may also be provided according to the device such as wrist-worn devices, chest-strapped devices, head worn devices, ankle worn devices, neck worn devices or any other form of body mountable device.

As discussed above, certain embodiments make use of a controller. The controller can be implemented in numerous ways, with software and/or hardware, to perform the various functions required. A processor is one example of a controller which employs one or more microprocessors that may be programmed using software (e.g., microcode) to perform the required functions. A controller may however be implemented with or without employing a processor, and also may be implemented as a combination of dedicated hardware to perform some functions and a processor (e.g., one or more programmed microprocessors and associated circuitry) to perform other functions.

Examples of controller components that may be employed in various embodiments of the present disclosure include, but are not limited to, conventional microprocessors, application specific integrated circuits (ASICs), and field-programmable gate arrays (FPGAs).

In various implementations, a processor or controller may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor or controller.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. A body-mountable sensor unit (12), comprising:
a skin contact surface (16) for engaging with skin of a user when the unit is in a body-mounted configuration;
a PPG sensor, comprising at least one light source (20a, 20b), adapted to generate light of a particular spectral composition, and an optical detector (22), sensitive to at least a portion of said spectral composition, the at least one light source and optical detector arranged to respectively emit light to and receive light from the skin in contact with the skin-contact surface; and
an ECG sensor, the ECG sensor comprising at least one electrode (32a, 32b), being electrically exposed at the skin-contact surface;
a section of the skin contact surface (16) being defined by a skin contact surface element (28), the skin contact surface element (28) having at least a portion arranged between the optical detector (22) and the at least one light source (20a, 20b) of the PPG sensor, wherein said skin contact surface element (28) is adapted to be absorptive of at least a majority of said spectral composition, and
wherein a first electrode (32a) of said at least one electrode (32a, 32b) is located at, or forms in, at least a part of said skin contact surface element (28), the first electrode (32a) being adapted to be absorptive of at least a majority of said spectral composition.

2. A body-mountable sensor unit (12) as claimed in claim 1, wherein the skin contact surface element (28) and/or the first electrode (32a) is black or grey in color.

3. A body-mountable sensor unit (12) as claimed in claim 1 or 2, wherein said first electrode (32a) is formed of a material comprising:
a conductive silicone material configured to enable uptake or diffusion of moisture from the skin of a user in which the electrode is placed in contact,
electrically conductive particles, the particles including at least electrically conductive carbon particles, and
a detergent configured to facilitate a flow of ions through the conductive silicone material.

4. A body-mountable sensor unit (12) as claimed in any of claims 1-3, wherein the first electrode (32a) is located at, or forms at least a part of, said at least a portion of the skin contact surface element (28).

5. A body-mountable sensor unit (12) as claimed in any of claims 1-4, wherein said at least portion of the skin contact surface element (28) spans a distance between the at least one optical detector (22) and the at least one light source (20a, 20b).

6. A body mountable sensor unit as claimed in any of claims 1-5, wherein the skin contact surface element (28) extends away from the optical detector (22) to a point or a line between the at least one light source (20a, 20b) and a periphery of the skin contact surface (16).

7. A body-mountable sensor unit (12) as claimed in any of claims 1-6, wherein the skin contact surface element (28) is an annular surface element, arranged extending around at least the optical detector (22) and/or the at least one light source (20a, 20b).

8. A body-mountable sensor unit (12) as claimed in any of claims 1-7, wherein the unit further comprises a light-blocking ring (30) surrounding at least the at least one light source (20a, 20b) and optical detector (22), the light-blocking ring (30) protruding from the skin contact surface (16) for engaging with skin of a user in use to block passage of ambient light.

9. A body-mountable sensor unit (12) as claimed in claim 8, wherein the light-blocking ring (30) extends around a peripheral region of the skin contact surface (16).

10. A body-mountable sensor unit (12) as claimed in claim 8 or 9, wherein the light-blocking ring (30) carries at least a further electrode (32b) of the at least one electrode (32a, 32b) of the ECG sensor, the further electrode (32b) being electrically exposed at a skin-engaging surface of the light-blocking ring (30).

11. A body-mountable sensor unit (12) as claimed in any of claims 1-10, wherein the optical detector (22) is disposed in a central region of the skin contact surface (16), and the at least one light source (20a, 20b) is disposed at a location between the optical detector (22) and a periphery of the skin contact surface (16).

12. A body-mountable sensor unit (12) as claimed in any of claims 1-11, wherein the sensor unit includes a plurality of light sources (20a, 20b), arranged around the optical detector (22), the at least portion of the skin contact surface element (28) extending annularly around the optical detector (22), and including a portion between each of the light sources (20a, 20b) and the optical detector (22).

13. A body-mountable sensor unit (12) as claimed in any of claims 1-12, wherein the body-mountable sensor unit is in the form of a body-mountable or wearable patch.

14. A body-mountable sensor unit (12) as claimed in any of claims 1-12, wherein the body-mountable sensor unit is the form of a wearable device, for example a wrist-worn device.

15. A body-mountable sensor unit (12) as claimed in any of claims 1-14, further comprising a controller arranged to receive signal outputs from the at least one electrode (32a, 32b) and from the optical detector (22), and to derive a measure of one or more physiological parameters based on the signal outputs.

## Patentansprüche

1. Am Körper montierbare Sensoreinheit (12), umfassend:
eine Hautkontaktfläche (16) zum Eingriff mit der Haut eines Benutzers, wenn sich die Einheit in einer am Körper montierten Konfiguration befindet;
einen PPG-Sensor, der mindestens eine Lichtquelle (20a, 20b) umfasst, die geeignet ist, um Licht einer bestimmten spektralen Zusammensetzung zu erzeugen, und einen optischen Detektor (22), der für mindestens einen Teil der spektralen Zusammensetzung empfindlich ist, wobei die mindestens eine Lichtquelle und der optische Detektor so angeordnet sind, dass sie jeweils Licht an die Haut emittieren und Licht von ihr empfangen, die mit der Hautkontaktfläche in Kontakt steht; und
einen EKG-Sensor, wobei der EKG-Sensor mindestens eine Elektrode (32a, 32b) umfasst, die an der Hautkontaktfläche elektrisch freigelegt ist;
wobei ein Abschnitt der Hautkontaktfläche (16) durch ein Hautkontaktflächenelement (28) definiert ist, wobei das Hautkontaktflächenelement (28) mindestens einen Abschnitt aufweist, der zwischen dem optischen Detektor (22) und der mindestens einen Lichtquelle (20a, 20b) des PPG-Sensors angeordnet ist, wobei das Hautkontaktflächenelement (28) angepasst ist, um mindestens einen Großteil der spektralen Zusammensetzung zu absorbieren, und
wobei eine erste Elektrode (32a) der mindestens einen Elektrode (32a, 32b) an mindestens einem Abschnitt des Hautkontaktflächenelements (28) angeordnet ist oder darin ausgebildet ist, wobei die erste Elektrode (32a) angepasst ist, um mindestens einen Großteil der spektralen Zusammensetzung zu absorbieren.

2. Am Körper montierbare Sensoreinheit (12) nach Anspruch 1, wobei das Hautkontaktoberflächenelement (28) und/oder die erste Elektrode (32a) eine schwarze oder graue Farbe aufweist.

3. Am Körper montierbare Sensoreinheit (12) nach Anspruch 1 oder 2, wobei die erste Elektrode (32a) aus einem Material gebildet ist, umfassend:
ein leitfähiges Silikonmaterial, das konfiguriert ist, um die Aufnahme oder Diffusion von Feuchtigkeit von der Haut eines Benutzers zu ermöglichen, mit der die Elektrode in Kontakt gebracht wird,
elektrisch leitfähige Partikel, wobei die Partikel zumindest elektrisch leitfähige Kohlenstoffpartikel umfassen, und
ein Reinigungsmittel, das konfiguriert ist, um einen Ionenfluss durch das leitfähige Silikonmaterial zu erleichtern.

4. Am Körper montierbare Sensoreinheit (12) nach einem der Ansprüche 1 bis 3, wobei die erste Elektrode (32a) an dem mindestens einen Abschnitt des Hautkontaktflächenelements (28) angeordnet ist oder mindestens einen Teil davon bildet.

5. Am Körper montierbare Sensoreinheit (12) nach einem der Ansprüche 1 bis 4, wobei der mindestens eine Abschnitt des Hautkontaktoberflächenelements (28) einen Abstand zwischen dem mindestens einen optischen Detektor (22) und der mindestens einen Lichtquelle (20a, 20b) überspannt.

6. Am Körper montierbare Sensoreinheit nach einem der Ansprüche 1 bis 5, wobei sich das Hautkontaktflächenelement (28) von dem optischen Detektor (22) weg zu einem Punkt oder einer Linie zwischen der mindestens einen Lichtquelle (20a, 20b) und einer Peripherie der Hautkontaktfläche (16) erstreckt.

7. Am Körper montierbare Sensoreinheit (12) nach einem der Ansprüche 1 bis 6, wobei das Hautkontaktflächenelement (28) ein ringförmiges Flächenelement ist, das sich um mindestens den optischen Detektor (22) und/oder die mindestens eine Lichtquelle (20a, 20b) erstreckend angeordnet ist.

8. Am Körper montierbare Sensoreinheit (12) nach einem der Ansprüche 1 bis 7, wobei die Einheit ferner einen lichtblockierenden Ring (30) umfasst, der mindestens die mindestens eine Lichtquelle (20a, 20b) und den optischer Detektor (22) umgibt, wobei der lichtblockierende Ring (30) von der Hautkontaktfläche (16) vorsteht, um mit der Haut eines Benutzers im Gebrauch in Eingriff zu kommen, um den Durchgang von Umgebungslicht zu blockieren.

9. Am Körper montierbare Sensoreinheit (12) nach Anspruch 8, wobei sich der lichtblockierende Ring (30) um einen peripheren Bereich der Hautkontaktfläche (16) herum erstreckt.

10. Am Körper montierbare Sensoreinheit (12) nach Anspruch 8 oder 9, wobei der lichtblockierende Ring (30) mindestens eine weitere Elektrode (32b) der mindestens einen Elektrode (32a, 32b) des EKG-Sensors trägt, wobei die weitere Elektrode (32b) an einer Hauteingriffsfläche des lichtblockierenden Rings (30) elektrisch freigelegt ist.

11. Am Körper montierbare Sensoreinheit (12) nach einem der Ansprüche 1 bis 10, wobei der optische Detektor (22) in einem mittleren Bereich der Hautkontaktfläche (16) angeordnet ist und das mindestens eine Lichtquelle (20a, 20b) an einer Stelle zwischen dem optischen Detektor (22) und einer Peripherie der Hautkontaktfläche (16) angeordnet ist.

12. Am Körper montierbare Sensoreinheit (12) nach einem der Ansprüche 1 bis 11, wobei die Sensoreinheit eine Vielzahl von Lichtquellen (20a, 20b) umfasst, die um den optischen Detektor (22) herum angeordnet sind, wobei sich zumindest ein Abschnitt des Hautkontaktflächenelements (28) ringförmig um den optischen Detektor (22) erstreckt und einen Abschnitt zwischen jeder der Lichtquellen (20a, 20b) und dem optischen Detektor (22) enthält.

13. Am Körper montierbare Sensoreinheit (12) nach einem der Ansprüche 1 bis 12, wobei die am Körper montierbare Sensoreinheit in Form eines am Körper montierbaren oder tragbaren Pflasters vorliegt.

14. Am Körper montierbare Sensoreinheit (12) nach einem der Ansprüche 1 bis 12, wobei die am Körper montierbare Sensoreinheit die Form einer tragbaren Vorrichtung hat, beispielsweise einer am Handgelenk getragenen Vorrichtung.

15. Am Körper montierbare Sensoreinheit (12) nach einem der Ansprüche 1 bis 14, die ferner eine Steuerung umfasst, die angeordnet ist, um Signalausgaben von der mindestens einen Elektrode (32a, 32b) und von dem optischen Detektor (22) zu empfangen, und um basierend auf den Signalausgaben ein Maß für einen oder mehrere physiologische Parameter abzuleiten.

## Revendications

1. Unité de capteur pouvant être montée sur un corps (12), comprenant:
une surface de contact avec la peau (16) destinée à venir en contact avec la peau d'un utilisateur lorsque l'unité est dans une configuration montée sur le corps;
un capteur PPG, comprenant au moins une source de lumière (20a, 20b), adaptée pour générer une lumière d'une composition spectrale particulière, et un détecteur optique (22), sensible à au moins une partie de ladite composition spectrale, l'au moins une source de lumière et un détecteur optique étant agencés pour respectivement émettre de la lumière vers et recevoir de la lumière depuis la peau en contact avec la surface de contact avec la peau; et
un capteur ECG, le capteur ECG comprenant au moins une électrode (32a, 32b), qui est électriquement exposée au niveau de la surface de contact avec la peau;
une section de la surface de contact avec la peau (16) étant définie par un élément de surface de contact avec la peau (28), l'élément de surface de contact avec la peau (28) ayant au moins une partie agencée entre le détecteur optique (22) et l'au moins une source de lumière (20a, 20b) du capteur PPG, où ledit élément de surface de contact avec la peau (28) est adapté pour absorber au moins une majorité de ladite composition spectrale, et
où une première électrode (32a) de ladite au moins une électrode (32a, 32b) est située sur, ou est formée dans, au moins une partie dudit élément de surface de contact avec la peau (28), la première électrode (32a) étant adaptée pour absorber au moins une majorité de ladite composition spectrale.

2. Unité de capteur pouvant être montée sur le corps (12) telle que revendiquée dans la revendication 1, dans laquelle l'élément de surface de contact avec la peau (28) et/ou la première électrode (32a) est de couleur noire ou grise.

3. Unité de capteur pouvant être montée sur le corps (12) telle que revendiquée dans la revendication 1 ou 2, dans laquelle ladite première électrode (32a) est formée d'un matériau comprenant:
un matériau en silicone conducteur configuré pour permettre l'absorption ou la diffusion de l'humidité de la peau d'un utilisateur dans lequel l'électrode est mise en contact,
des particules électriquement conductrices, les particules comprenant au moins des particules de carbone électriquement conductrices, et
un détergent configuré pour faciliter un flux d'ions à travers le matériau en silicone conducteur.

4. Unité de capteur pouvant être montée sur le corps (12) telle que revendiquée dans l'une quelconque des revendications 1 à 3, dans laquelle la première électrode (32a) est située au niveau de, ou forme au moins une partie de, ladite au moins une partie de l'élément de surface de contact avec la peau (28).

5. Unité de capteur pouvant être montée sur le corps (12) telle que revendiquée dans l'une quelconque des revendications 1 à 4, dans laquelle ladite au moins une partie de l'élément de surface de contact avec la peau (28) couvre une distance entre l'au moins un détecteur optique (22) et l'au moins une source de lumière (20a, 20b).

6. Unité de capteur pouvant être montée sur le corps telle que revendiquée dans l'une quelconque des revendications 1 à 5, dans laquelle l'élément de surface de contact avec la peau (28) s'étend à distance du détecteur optique (22) jusqu'à un point ou une ligne entre l'au moins une source de lumière (20a, 20b) et une périphérie de la surface de contact avec la peau (16).

7. Unité de capteur pouvant être montée sur le corps (12) telle que revendiquée dans l'une quelconque des revendications 1 à 6, dans laquelle l'élément de surface de contact avec la peau (28) est un élément de surface annulaire, agencé de manière à s'étendre autour d'au moins le détecteur optique (22) et/ou de l'au moins une source de lumière (20a, 20b).

8. Unité de capteur pouvant être montée sur le corps (12) telle que revendiquée dans l'une quelconque des revendications 1 à 7, dans laquelle l'unité comprend en outre un anneau bloquant la lumière (30) entourant au moins l'au moins une source de lumière (20a, 20b) et l'au moins un détecteur optique (22), l'anneau bloquant la lumière (30) dépassant de la surface de contact avec la peau (16) pour venir en contact avec la peau d'un utilisateur lors de l'utilisation pour bloquer le passage de la lumière ambiante.

9. Unité de capteur pouvant être montée sur le corps (12) telle que revendiquée dans la revendication 8, dans laquelle l'anneau bloquant la lumière (30) s'étend autour d'une région périphérique de la surface de contact avec la peau (16).

10. Unité de capteur pouvant être montée sur le corps (12) telle que revendiquée dans la revendication 8 ou 9, dans laquelle l'anneau bloquant la lumière (30) porte au moins une autre électrode (32b) de l'au moins une électrode (32a, 32b) du capteur ECG, l'autre électrode (32b) étant électriquement exposée au niveau d'une surface venant en contact avec la peau de l'anneau bloquant la lumière (30).

11. Unité de capteur pouvant être montée sur le corps (12) telle que revendiquée dans l'une quelconque des revendications 1 à 10, dans laquelle le détecteur optique (22) est disposé dans une région centrale de la surface de contact avec la peau (16), et l'au moins une source de lumière (20a, 20b) est disposée à un emplacement entre le détecteur optique (22) et une périphérie de la surface de contact avec la peau (16).

12. Unité de capteur pouvant être montée sur le corps (12) telle que revendiquée dans l'une quelconque des revendications 1 à 11, dans laquelle l'unité de capteur comprend une pluralité de sources de lumière (20a, 20b), agencées autour du détecteur optique (22), l'au moins une partie de l'élément de surface de contact avec la peau (28) s'étendant de manière annulaire autour du détecteur optique (22), et comprenant une partie entre chacune des sources de lumière (20a, 20b) et le détecteur optique (22).

13. Unité de capteur pouvant être montée sur le corps (12) telle que revendiquée dans l'une quelconque des revendications 1 à 12, dans laquelle l'unité de capteur pouvant être montée sur le corps se présente sous la forme d'un patch qui peut être monté sur le corps ou porté.

14. Unité de capteur pouvant être montée sur le corps (12) telle que revendiquée dans l'une quelconque des revendications 1 à 12, dans laquelle l'unité de capteur pouvant être montée sur le corps se présente sous la forme d'un dispositif qui peut être porté, par exemple un dispositif porté au poignet.

15. Unité de capteur pouvant être montée sur le corps (12) telle que revendiquée dans l'une quelconque des revendications 1 à 14, comprenant en outre un dispositif de commande agencé pour recevoir des sorties de signal provenant de l'au moins une électrode (32a, 32b) et du détecteur optique (22), et pour dériver une mesure d'un ou plusieurs paramètres physiologiques sur la base des sorties de signal.
